(19) Europäisches Patentamt / European Patent Office / Office européen des brevets

(11) EP 1 470 133 B1

(12) # EUROPEAN PATENT SPECIFICATION

(45) Date of publication and mention of the grant of the patent:
**10.09.2008 Bulletin 2008/37**

(21) Application number: **03707635.3**

(22) Date of filing: **29.01.2003**

(51) Int Cl.:
***C07F 5/02*** (2006.01) ***C12Q 1/54*** (2006.01)

(86) International application number:
**PCT/US2003/002915**

(87) International publication number:
**WO 2003/064434 (07.08.2003 Gazette 2003/32)**

(54) **ELECTROCHEMICAL SACCHARIDE SENSOR**

ELEKTROCHEMISCHER SACCHARIDSENSOR

DETECTEUR ELECTROCHIMIQUE DE SACCHARIDES

(84) Designated Contracting States:
**CH DE FR GB IT LI**

(30) Priority: **30.01.2002 US 60790**

(43) Date of publication of application:
**27.10.2004 Bulletin 2004/44**

(73) Proprietor: **Beckman Coulter, Inc.**
**Fullerton,**
**California 92834-3100 (US)**

(72) Inventors:
- **JAMES, Tony, D.**
  **Radstock, Bath and South East Somerset B (GB)**
- **ARIMORI, Susumu**
  **Iizuka, Fukuoka 820-0011 (JP)**

(74) Representative: **Müller-Boré & Partner**
**Patentanwälte**
**Grafinger Strasse 2**
**81671 München (DE)**

(56) References cited:
**US-A- 5 503 770** **US-B1- 6 387 672**

- **ARIMORI, SUSUMU ET AL: "A modular electrochemical sensor for saccharides" CHEMICAL COMMUNICATIONS (CAMBRIDGE, UNITED KINGDOM) , (20), 2368-2369 CODEN: CHCOFS; ISSN: 1359-7345, 2002, XP002375696**
- **AIME, SILVIO ET AL: "Synthesis and characterization of a novel DTPA-like gadolinium (III) complex: a potential reagent for the determination of glycated proteins by water proton NMR relaxation measurements" INORGANIC CHEMISTRY , 32(10), 2068-71 CODEN: INOCAJ; ISSN: 0020-1669, 1993, XP002375697**
- **DATABASE CAPLUS [Online] (COLUMBUS OH, USA) MURAKAMI ET AL.: 'Sugar sensing utilizing aggregation properties of boronic-acid-appended porphyrins and metalloporphyrins', XP002964845 Retrieved from STN Database accession no. 1994-548059 & JOURNAL OF THE CHEMICAL SOCIETY, PERKIN TRANSACTIONS no. 5, 1994, pages 975 - 981**
- **DATABASE CAPLUS [Online] (COLUMBUS OH, USA) MIZUNO ET AL.: 'Re-investigation of optical sensing properties of boronic-acid-appended ReI complexes for saccharides', XP002964846 Retrieved from STN Database accession no. 2000-74450 & PERKIN vol. 1, no. 3, 2000, pages 407 - 413**
- **DATABASE CAPLUS [Online] (COLUMBUS OH, USA) TAKEUCHI ET AL.: 'Chirality sensing saccahrides wusing a boronic acid-appended chiral ferrocene derivative', XP002964847 Retrieved from STN Database accession no. 2000-698599 & TETRAHEDRON: ASYMMETRY vol. 11, no. 16, 2000, pages 3311 - 3322**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 470 133 B1

## Description

The following description provides a summary of information relevant to the present invention and is not a concession that any of the information provided or publications referenced herein is prior art to the presently claimed invention.

Saccharides, more commonly known as sugars, are organic compounds that play important roles in living organisms. Of particular medical and clinical interest is the monosaccharide D-glucose, which is a critical biochemical energy source for cells. Glucose is stored in the liver as glycogen, and is released as needed for energy consumption. The production and the consumption of glucose are regulated such that the concentration of glucose is relatively constant in the body fluids of a normal or healthy mammals. A disruption of this regulation of glucose can be associated with diseases such as diabetes and adrenal insufficiency in humans.

The detection of glucose in the blood or the urine provides valuable information for the diagnosis of these diseases. Electrochemical detection of saccharides by enzymatic decomposition of saccharides is known. One particular assay utilizes the enzyme glucose oxydase, which decomposes glucose to release hydrogen peroxide. The hydrogen peroxide can be measured by an electrode. A problem with this assay is that the enzymatic activity of the naturally occurring enzyme degrades with time. Thus the sample cannot be recycled for repeated assays because the glucose and enzyme have degraded. Further, this susceptibility to degradation makes the assay vulnerable to imprecision.

Recent approaches to detect saccharides involve the use of synthetic molecular receptors that bind to saccharides and exhibit a non-enzymatic chemically detectable change upon binding with the saccharide. Specifically, receptor molecules that incorporate boronic acid moieties have been shown to bind to saccharides through covalent interactions in aqueous basic media. The most common interaction is with cis-1,2- or 1,3-diols of saccharides to form five- or six-membered rings respectively.

Recently, a simple boronic acid receptor was reported that binds and detects sorbitol, fructose, and glucose by electrochemical changes. "Redox switching of carbohydrate binding to ferrocene boronic acid", A.N.J. Moore, D. D. M. Wayner, Canadian Journal of Chemistry-Revue Canadienne De Chimie, 77, 681 (1999). This monoboronic acid receptor exhibited selectivity for D-fructose. This is consistent with early reports that monoboronic acids have an inherent selectivity for D-fructose. "Polyol complexes and structure of the benzeneboronate ion" J. P. Lorand, J. D. Edward, Journal of Organic Chemistry 1959, 24, 769.

Although glucose may be detected electrochemically through the above receptor, the receptor is not selective for glucose and the presence of other saccharides will interfere with any assay utilizing such a receptor. Unfortunately, the most common glucose measurement applications use complex samples such as plasma and the large number of interfering compounds make electrochemical determinations very difficult if not impossible. What is needed is a selective molecular receptor system for detecting glucose having the specificity required for clinical assays of complex samples.

US 6,387,672 A describes a modular fluorescence sensor comprising binding groups, an aliphatic spacer an anchoring group for immobilizing said sensor on a solid substrate, as well a methods for its synthesis and uses thereof for labelling solid substrates.

The invention satisfies this need. The invention provides compounds for detecting saccharides by electrochemistry. The compounds comprise a first boronic acid group attached by a first linker group to a first amine group, where the first amine group is attached to a reporter group comprising an organometalic reporter moiety; a second boronic acid group attached by a second linker group to a second amine group, where the second amine group is attached to an R group; and a spacer group attaching the first amine group to the second amine group. Optionally, the first linker group is an aromatic ring, a heteroaromatic ring, an alkyl group, an alkene group, or an alkyne groups; the spacer group is an alkyl group, more specifically a hexamethylene group; the R group is a hydrogen, an akyl group, an aryl group, or a reporter group comprising an organometallic reporter moiety, more specifically ferrocene or a ferrocene derivative; the second linker group is an aromatic ring, a heteroaromatic ring, an alkyl group, an alkene group, or an alkyne group.

The compounds of the invention are further represented by the structural formula:

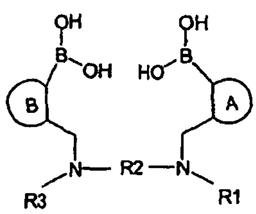

where A is a first linker group, which is an aromatic ring or a heteroaromatic ring; R1 is a reporter group comprising an

organometallic reporter group; R2 is a linker group, which is an alkyl group or an aryl group; R3 is an R group, which is hydrogen, an alkyl group, an aryl group, or an organometallic reporter group; and B is the second linker group, which is an aromatic ring or heteroaromatic ring.

More specifically, compounds of the invention are represented by the structural formula:

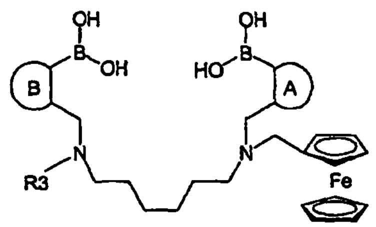

where A, B, and R3 are defined above.

In an embodiment of the invention, the compounds have a higher stability constant for glucose than for other saccharides. More specifically, the compounds have a relative stability constant for glucose that is at least two times higher, and preferably between about two and about fifteen times higher than the relative stability constant for the compound and other saccharides.

The invention includes a method for detecting saccharides in a sample comprising providing a predetermined amount of a compound of the invention; treating the sample with the compound; and detecting saccharides bound to the compound. In a preferred embodiment, the compound has the formula below and/or the method for detecting saccharides preferentially detects glucose relative to other saccharides.

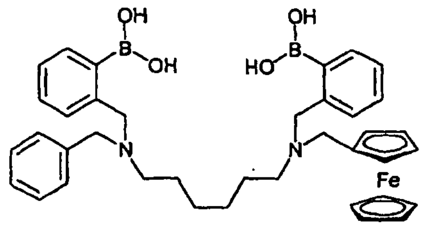

These features, aspects and advantages of the present invention will become better understood with regard to the following description, appended claims and accompanying figures where:

Figure 1 illustrates a method of preparing an electrochemical glucose-sensing compound according to the invention;

Figure 2 illustrates a method of preparing an electrochemical saccharide-sensing compound;

Figure 3 illustrates a method of preparing a saccharide-sensing compound;

Figure 4 illustrates a Differential Pulse Voltammogram (DPV) plot of compound 1 in the presence of concentrations of glucose ranging from 0-0.1 mol $dm^{-3}$. The D-glucose concentration for each of the curves from top to bottom are the following: 0 / mol $dm^{-3}$ (curve 1), $1.11x10^{-4}$ / mol $dm^{-3}$ (curve 2), $3.89x10^{-4}$ /mol $dm^{-3}$ (curve 3), $6.66x10^{-4}$ /mol $dm^{-3}$ (curve 4), $1.04x10^{-3}$ /mol $dm^{-3}$ (curve 5), $2.04x10^{-3}$ /mol $dm^{-3}$ (curve 6), $3.79x10^{-3}$ /mol $dm^{-3}$ (curve 7), $6.01x10^{-3}$ /mol $dm^{-3}$. (curve 8), $8.18x10^{-3}$ /mol $dm^{-3}$ (curve 9), $1.03x10^{-2}$ /mol $dm^{-3}$ (curve 10), $2.03x10^{-2}$ /mol $dm^{-3}$ (curve 11), $3.96x10^{-2}$ /mol $dm^{-3}$ (curve 12), $6.19x10^{-2}$ /mol $dm^{-3}$ (curve 13), $8.31x10^{-2}$ /mol $dm^{-3}$ (curve 14), and $1.0x10^{-1}$ / mol $dm^{-3}$ (curve 15).

Figure 5 illustrates a Differential Pulse Voltammetry spectra of compound 4 and compound 6 in the presence of increasing concentrations of glucose. The D-glucose curves were not numbered due to their proximity. The concentration for each of the curves from top to bottom are the following: 0 / mol $dm^{-3}$, $1.11x10^{-4}$ /mol $dm^{-3}$, $4.07x10^{-4}$ / mol $dm^{-3}$, $6.66x10^{-4}$ /mol $dm^{-3}$, $1.02x10^{-3}$ /mol $dm^{-3}$, $2.15x10^{-3}$ /mol $dm^{-3}$, $4.27x10^{-3}$ /mol $dm^{-3}$, $6.33x10^{-3}$ / mol $dm^{-3}$, $8.31x10^{-3}$ / mol dm-3, $1.02x10^{-2}$ / mol $dm^{-3}$, $2.00x10^{-2}$ / mol $dm^{-3}$, $3.90x10^{-2}$ / mol $dm^{-3}$, $5.97x10^{-2}$ / mol $dm^{-3}$, $7.91.x10^{-2}$ / mol $dm^{-3}$, $1.02x10^{-1}$ / mol $dm^{-3}$;

Figure 6 illustrates a plot of relative current *vs*. saccharide concentration for compound 1 ($5.0x10^{-5}$ mol $dm^{-3}$ each) for glucose, galactose, fructose, and mannose measured in 52.1wt% MeOH at pH 8.21 (phosphate buffer); and

Figure 7 illustrates a plot of relative current *vs*. saccharide concentration for compound 4 and compound 6 ($5.0x10^{-5}$ mol $dm^{-3}$) for glucose, galactose, fructose, and mannose measured in 52.1wt% MeOH at pH 8.21 (phosphate buffer).

The following discussion describes embodiments of the invention and several variations of these embodiments. This discussion should not be construed, however, as limiting the invention to those particular embodiments. Practitioners skilled in the art will recognize numerous other embodiments as well. In all of the embodiments described herein that are referred to as being preferred or particularly preferred, these embodiments are not essential even though they may be preferred.

Structure of Glucose Sensing Compounds

The compounds of the invention are useful for detecting saccharrides, and in particular for detecting glucose. In preferred embodiments, the chemical structure of the compounds is such that they selectively detect glucose because they form complexes with glucose that have a higher stability constant than complexes formed with other saccharides. This glucose selectivity is accomplished by providing diboronic acid compounds and systems that control saccharide selectivity through two point binding.

The compounds of the invention comprise a first boronic acid group that is attached by a first linker group to a first tertiary amine. The first tertiary amine is attached to a reporter group comprising an organometalic reporter moiety. The first tertiary amine is additionally attached by a spacer group to a second tertiary amine. The second tertiary amine is attached to an R group and a spacer group wherein the R group comprises hydrogen, an akyl, an aryl, or a reporter group comprising an organometalic reporter moiety. The spacer group is attached to a second boronic acid group.

In typical embodiments, the first linker comprises an aromatic ring, heteroaromatic ring, alkyl group, alkene group, or alkyne group. The presence of an amine group proximal to the boronic acid facilitates the binding of the compound to saccharides at a relatively a neutral pH. The interaction of a boronic acid (Lewis acid) and neighboring tertiary amine (Lewis base) is strengthened on saccharide binding. Conversely, simple boronic acids typically only bind saccharides at a high pH.

Although the spacer group may comprises a straight, branched, or cyclic structure, in the preferred embodiment the spacer is a straight chained alkyl group. Typically the spacer group comprises 1 to 10 carbon atoms, but spacers of larger length may also be used to match the size of various desired analytes. In a preferred embodiment, the number of carbons is 6 and the spacer group comprises hexamethylene. This compound has a higher stability constant with glucose than for other saccharides that enables the compound to selectively detect glucose.

Preferably, the second linker group comprises an aromatic ring, heteroaromatic ring, alkyl group, alkene group, or alkyne group. In a preferred embodiment, the organometalic reporter moiety comprises ferrocene or a ferrocene derivative.

Typical embodiments of a compound falling within the chemical structure of the present invention can be expressed by the general formula:

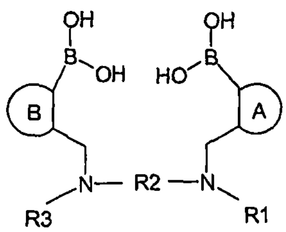

where the first linker group (A) comprises an aromatic ring or heteroaromatic ring; the reporter group (R1) comprises an organometalic reporter group; the spacer group (R2) comprises an akyl or an aryl; R3 comprises hydrogen, an akyl, an aryl, or a organometalic reporter group; and the second linker group (B) comprises an aromatic ring or heteroaromatic ring. Preferred embodiments have one or more of the following specific chemical groups: the spacer group (R2) is an alkyl group; the R1 and R3 groups are independently selected from the group consisting of hydrogen, an akyl, an aryl, and a reporter group comprising an organometalic reporter moiety; the organometalic reporter moiety comprises ferrocene or a ferrocene derivative. In a further preferable embodiment, the spacer group (R2) comprises hexamethylene.

Embodiments of compounds falling within the chemical structure of the present invention can further be expressed by the general formula:

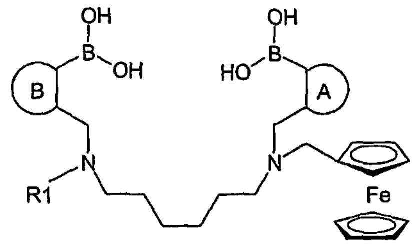

where A represents an aromatic ring or heteroaromatic ring; R1 is selected from the group consisting of hydrogen, an akyl, an aryl, and a organometalic reporter group; and B is an aromatic ring or heteroaromatic ring. This compound has a higher stability constant for glucose than for other saccharides that confers a selective detection of glucose relative to other sacharides.

In a particularly preferred embodiment the compound comprises the chemical formula (1), and again the compound has a higher stability constant for glucose than for other saccharides that allows it to selectively detect glucose. The preparation of compound 1, referred to herein as N-benzyl-N, N'-bis (2-boronobenzyl)-N'-ferrocenylmethyl hexamethyl-1, 6-diamine, is illustrated in Figure 1 and is explained in detail in Examples I, II, and III.

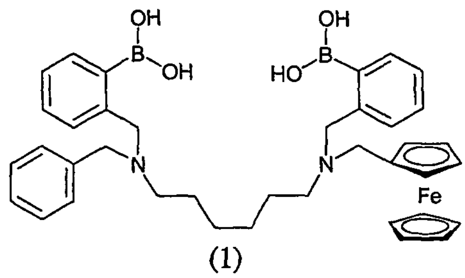

(1)

**Methods for Detecting Glucose and Determining Stability Constants**

The glucose in the sample is detected by determining glucose sensing compound bound to glucose. The redox potential of the organometalic reporter moiety of compound changes upon binding to a saccharide.

Redox potentials can be measured using a variety of standard electrochemical techniques such as cyclic voltammogram (CV) and pulse Voltammetry, including Tast polarography, Normal pulse voltammetry (NPV), Differential pulse voltammetry (DPV), Square-wave voltammery (SWV), Reverse pulse Voltammetry (RPV), Differential normal pulse voltammetry (DNPV), and Double differential pulse voltammetry (DDPV). These techniques are well known in the art. See for example C. M. A. Brett and A. M. Oliviera Brett, "Electrochemistry", Oxford university press, 1993, incorporated herein by reference. Electrochemical detection by Differential Pulse Voltammetry (DPV) is described in detail in Example VIII. Figure 4 illustrates Differential Pulse Voltammogram (DPV) curves of compound 1 in the presence of different concentrations of D-glucose.

In order to investigate binding selectivity of compound 1 with various saccharides, the stability constant of compound 1 with particular saccharides was compared to the stability constant of monoboronic acids that are relatively non-specific with the same saccharides. The non-specific monobornic acids illustrated below were used for determining and comparing the relative binding affinity of the glucose sensing compound to glucose and other saccharides.

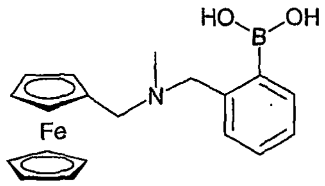

(4)

The preparation of compound 4, referred to herein as N- (2-borinobenzyl)-N-methyl ferrocenyl methylamine (4), is illustrated in Figure 2 and is explained in detail in the Example V.

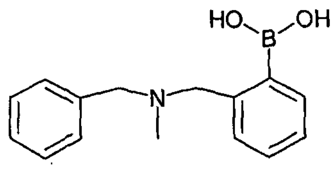

(6)

The preparation of compound 6, referred to herein as N- (2-borinobenzyl)-N-methyl benzylamine 6, is illustrated in Figure 3 and is explained in detail in the Example VII.

Figure 5 illustrates Differential Pulse Voltammetry curves of compound 4 and compound 6 in the presence of increasing concentrations of glucose. Figures 4 and 5 show that as the amount of saccharide increases the relative current indicated by the amplitude of curve decreases. It is apparent from these two Figures that the observed changes in current in response to higher concentrations of glucose is greater in compound 1 shown in Figure 4 than in compounds 4 and 6, shown in Figure 5.

In Figure 4, the glucose concentration {D-glucose} from top curve to bottom curve are as follows: 0 / mol $dm^{-3}$ (curve 1), $1.11x10^{-4}$ / mol $dm^{-3}$ (curve 2), $3.89x10^{-4}$ / mol $dm^{-3}$ (curve 3), $6.66x10^{-4}$ / mol $dm^{-3}$ (curve 4), $1.04x10^{-3}$ / mol $dm^{-3}$ (curve 5), $2.04x10^{-3}$ / mol $dm^{-3}$ (curve 6), $3.79x10^{-3}$ / mol $dm^{-3}$ (curve 7), $6.01x10^{-3}$ / mol $dm^{-3}$ (curve 8), $8.18x10^{-3}$ / mol $dm^{-3}$ (curve 9), $1.03x10^{-2}$ / mol $dm^{-3}$ (curve 10), $2.03x10^{-2}$ / mol $dm^{-3}$ (curve 11), $3.96x10^{-2}$ / mol $dm^{-3}$ (curve 12), $6.19x10^{-2}$ / mol $dm^{-3}$ (curve 13), $8.31x10^{-2}$ / mol $dm^{-3}$ (curve 14), and $1.0x10^{-1}$ / mol $dm^{-3}$ (curve 15). In Figure 5, the glucose concentration {D-glucose} from top curve to bottom curve are as follows: 0, $1.11\times10^{-4}$ $4.07\times10^{-4}$ $6.66\times10^{-4}$ $1.02\times10^{-3}$, $2.15\times10^{-3}$, $4.27\times10^{-3}$, $6.33\times10^{-3}$, $8.31\times10^{-3}$, $1.02\times10^{-2}$, $2.00\times10^{-2}$, $3.90\times10^{-2}$, $5.97\times10^{-2}$, $7.91\times10^{-2}$, $1.02\times10^{-1}$ /mol $dm^{-3}$. This data indicates that compound 1 is more sensitive to changing glucose concentrations than compounds 4 and 6.

Figure 6 is a plot of relative current to saccharide concentration for glucose, galactose, fructose, and mannose, in the presence of compound 1. Figure 7 is a plot of relative current to saccharide concentration for glucose, galactose, fructose, and mannose, in the presence of compounds 4 and 6. The data for Figure 6 and Figure 7 are summarized in Table I and Table II below.

Table I - Plot data for Figure 6

| {D-glucose} / mol $dm^{-3}$ | Relative current @ 0.357 V | {D-galactose} / mol $dm^{-3}$ | Relative current @ 0.357 V | {D-fructose} / mol $dm^{-3}$ | Relative current @ 0.357 V | {D-mannose} / mol $dm^{-3}$ | Relative current @ 0.357 V |
|---|---|---|---|---|---|---|---|
| 0.00 | 1.000 | 0.00 | 1.000 | 0.00 | 1.000 | 0.00 | 1.000 |
| $1.11\times10^{-4}$ | 1.000 | $1.11\times10^{-4}$ | 1.000 | $1.11\times10^{-4}$ | 0.977 | $1.11\times10^{-4}$ | 0.992 |
| $4.07\times10^{-4}$ | 1.000 | $4.07\times10^{-4}$ | 0.992 | $4.07\times10^{-4}$ | 0.938 | $4.26\times10^{-4}$ | 0.992 |
| $6.66\times10^{-4}$ | 1.000 | $7.40\times10^{-4}$ | 0.984 | $7.22\times10^{-4}$ | 0.898 | $6.66\times10^{4-}$ | 0.992 |
| $1.02\times10^{-3}$ | 0.992 | $9.99\times10^{-4}$ | 0.977 | $1.02\times10^{-3}$ | 0.867 | $9.99\times10^{-4}$ | 0.984 |

(continued)

| {D-glucose} / mol dm$^{-3}$ | Relative current @ 0.357 V | {D-galactose} / mol dm$^{-3}$ | Relative current @ 0.357 V | {D-fructose} / mol dm$^{-3}$ | Relative current @ 0.357 V | {D-mannose} / mol dm$^{-3}$ | Relative current @ 0.357 V |
|---|---|---|---|---|---|---|---|
| $2.15\times10^{-3}$ | 0.984 | $2.00\times10^{-3}$ | 0.961 | $1.94\times10^{3-}$ | 0.805 | $1.94\times10^{-3}$ | 0.977 |
| $4.27\times10^{-3}$ | 0.961 | $3.83\times10^{-3}$ | 0.922 | $3.83\times10^{-3}$ | 0.717 | $3.87\times10^{-3}$ | 0.938 |
| $6.33\times10^{-3}$ | 0.945 | $6.01\times10^{-3}$ | 0.891 | $5.79\times10^{-3}$ | 0.672 | $5.88\times10^{-3}$ | 0.922 |
| $8.31\times10^{-3}$ | 0.921 | $7.86\times10^{-3}$ | 0.859 | $8.05\times10^{-3}$ | 0.637 | $8.03\times10^{-3}$ | 0.898 |
| $1.02\times10^{-2}$ | 0.906 | $1.01\times10^{-2}$ | 0.859 | $1.03\times10^{-2}$ | 0.613 | $1.02\times10^{-2}$ | 0.844 |
| $2.00\times10^{-2}$ | 0.843 | $2.04\times10^{-2}$ | 0.768 | $2.04\times10^{-2}$ | 0.572 | $2.05\times10^{-2}$ | 0.773 |
| $3.90\times10^{-2}$ | 0.763 | $4.05\times10^{-2}$ | 0.678 | $4.09\times10^{-2}$ | 0.549 | $3.93\times10^{-2}$ | 0.773 |
| $5.97\times10^{-2}$ | 0.724 | $5.94\times10^{-2}$ | 0.643 | $6.01\times10^{-2}$ | 0.535 | $5.78\times10^{-2}$ | 0.731 |
| $7.91\times10^{-2}$ | 0.686 | $8.14\times10^{-2}$ | 0.616 | $8.01\times10^{-2}$ | 0.527 | $7.93\times10^{-2}$ | 0.696 |
| $1.03\times10^{-1}$ | 0.608 | $1.02\times10^{-1}$ | 0.605 | $1.03\times10^{-1}$ | 0.528 | $1.03\times10^{-1}$ | 0.670 |

Table II - Plot data for Figure 7

| {D-glucose } / mol dm$^{-3}$ | Relative current @ 0.357 V | {D-galactose} / mol dm$^{-3}$ | Relative current @ 0.357 V | {D-fructose} / mol dm$^{-3}$ | Relative current @ 0.357 V | {D-mannose} / mol dm$^{-3}$ | Relative current @ 0.357 V |
|---|---|---|---|---|---|---|---|
| 0.00 | 1.000 | 0.00 | 1.000 | 0.00 | 1.000 | 0.00 | 1.000 |
| $1.11\times10^{-4}$ | 1.000 | $1.11\times10^{-4}$ | 1.000 | $1.11\times10^{-4}$ | 0.977 | $1.11\times10^{-4}$ | 0.992 |
| $4.07\times10^{-4}$ | 1.000 | $4.07\times10^{-4}$ | 0.992 | $4.07\times10^{-4}$ | 0.938 | $4.26\times10^{-4}$ | 0.992 |
| $6.66\times10^{-4}$ | 1.000 | $7.40\times10^{-4}$ | 0.984 | $7.22\times10^{-4}$ | 0.898 | $6.66\times10^{-4}$ | 0.992 |
| $1.02\times10^{-3}$ | 0.992 | $9.99\times10^{-4}$ | 0.977 | $1.02\times10^{-3}$ | 0.867 | $9.99\times10^{-4}$ | 0.984 |
| $2.15\times10^{-3}$ | 0.984 | $2.00\times10^{-3}$ | 0.961 | $1.94\times10^{-3}$ | 0.805 | $1.94\times10^{-3}$ | 0.977 |
| $4.27\times10^{-3}$ | 0.961 | $3.83\times10^{-3}$ | 0.922 | $3.83\times10^{-3}$ | 0.717 | $3.87\times10^{-3}$ | 0.938 |
| $6.33\times10^{-3}$ | 0.945 | $6.01\times10^{-3}$ | 0.891 | $5.79\times10^{-3}$ | 0.672 | $5.88\times10^{-3}$ | 0.922 |
| $8.31\times10^{-3}$ | 0.921 | $7.86\times10^{-3}$ | 0.859 | $8.05\times10^{-3}$ | 0.637 | $8.03\times10^{-3}$ | 0.898 |
| $1.02\times10^{-2}$ | 0.906 | $1.01\times10^{-2}$ | 0.859 | $1.03\times10^{-2}$ | 0.613 | $1.02\times10^{-2}$ | 0.844 |
| $2.00\times10^{-2}$ | 0.843 | $2.04\times10^{-2}$ | 0.768 | $2.04\times10^{-2}$ | 0.572 | $2.05\times10^{-2}$ | 0.773 |
| $3.90\times10^{-2}$ | 0.763 | $4.05\times10^{-2}$ | 0.678 | $4.09\times10^{-2}$ | 0.549 | $3.93\times10^{-2}$ | 0.773 |
| $5.97\times10^{-2}$ | 0.724 | $5.94\times10^{-2}$ | 0.643 | $6.01\times10^{-2}$ | 0.535 | $5.78\times10^{-2}$ | 0.731 |
| $7.91\times10^{-2}$ | 0.686 | $8.14\times10^{-2}$ | 0.616 | $8.01\times10^{-2}$ | 0.527 | $7.93\times10^{-2}$ | 0.696 |
| $1.03\times10^{-1}$ | 0.608 | $1.02\times10^{-1}$ | 0.605 | $1.03\times10^{-1}$ | 0.528 | $1.03\times10^{-1}$ | 0.670 |

The Differential Pulse Voltammograms of these compounds were used to calculate the stability constant for each compound with a specific saccharide. The stability constant K for the saccharide complexes was calculated according to the following equation.

$$I_c = (I_{c0} + I_{cf} \text{ x } K \text{ x } \{\text{guest}\}) \ / \ (1+ K \text{ x } \{\text{guest}\})$$

Where $I_{c0}$ is the initial (minimum) current intensity; $I_{cf}$ is the final (maximum) current intensity; $I_c$ is the current intensity

for a particular concentration of guest saccharide; Kis the stability constant of the receptor with the guest; {guest} is the concentration of the guest; described in Fery-Forgues, S. et al., "Ion-responsive fluorescent compounds. 1. Effect of cation binding on photophysical properties of a benzoxazione derivative linked to monoaza-15-crown-5", J. Phys. Chem., 1988, 92, 6233 - 6237, incorporated herein by reference. The 'guest' is the analyte of interest, which is the particular saccharide being examined.

The stability constant (K) curves were analyzed in KaleidaGraph using nonlinear (Levenberg-Marquardt algorithm) curve fitting of equations (1). The errors reported are the standard errors (±s/ N) obtained from the best fit. KaleidaGraph Version 3.08d for the Macintosh, Published by Synergy Software and Developed by Abelbeck Software (Reading, PA). A user defined curve fit (1 + m2 * ml * (M0)) / (1 + ml * (M0)) derived from equations (1) was used in all calculations. The Initial value of m1 (K) was set to 1 and the initial value of m2 ($I_{cf}$) was set to 0.9. The variable (M0) was {guest}. The allowable error was set to 0.001%. For all curves the coefficient of determination ($r^2$) was > 0.99.

Table III presents data including the stability constants (K) for complexes of two electrochemical sensors with particular saccharides, which are listed in column 1. Column two of Table III includes the absolute stability constants determined for compound 1 with glucose, galactose, fructose, and mannose. Column three of Table III includes the absolute stability constants determined for the non-selective intermolecular system of compounds 4 plus 6 with glucose, galactose, fructose, and mannose. The fourth column presents the relative stability constants, which are derived by dividing the absolute stability constant of compound 1 for each particular saccharide by the absolute stability constant for binding with compounds 4 plus 6 in the presence of the same saccharide. The data demonstrates that compound 1 has a relative stability constant for glucose that is at least two times higher in comparison with other saccharides. Also, the relative stability constant of compound 1 to glucose is typically between about 2 and about 15 times greater than the relative stability constant of compound 1 with the other saccharides presented in Table III. This higher stability constant confers the relative specificity of compound 1 for glucose.

Table III

| Saccharide | 1 *K* | 4+ 6 *K* | 1/ (4+6) *K* Relative |
|---|---|---|---|
| D-glucose | 684±54 (1.00) | 17±2 (1.00) | 40 |
| D-galactose | 781±72 (0.99) | 47±2 (1.00) | 17 |
| D-fructose | 1478±72 (1.00) | 362±5 (1.00) | 4 |
| D-mannose | 149±9 (1.00) | 54±8 (1.00) | 3 |

EXAMPLE I

Preparation of N-benzyl-hexane-1, 6-diamine (2)

A solution of benzaldehyde (1.0ml, 10.0mmol) in THF (50ml) was added with heating at reflux to a solution of 1,6-diaminohexane (5.81g, 50.0mmol) and p-toluene sulfonic acid (9.51g, 50.0mmol) in ethanol (50ml) and then it was heated to reflux for 3h under a nitrogen atmosphere. After cooling to room temperature, sodium borohydride (1.13g, 30.0mmol) was added to the solution and it was stirred at room temperature for 1h. The solvent was removed under reduced pressure and the residue dissolved in chloroform. The chloroform phase was washed with water, and dried over magnesium sulphate, the solvent was removed under reduced pressure. The residue was washed with water (25ml), and it dissolved in chloroform and washed again with water (2x50ml). Then dried over magnesium sulphate, and the solvent removed under reduced pressure to yield a yellow oil (1.61g, 78%).

$_H$ (300MHz, $CDCl_3$, $Me_4Si$) : 1.1-1.5 (8H, m, $(CH_2)_4$), 2.55 (2H, t, NHC$H_2$), 2.65 (2H, t, ArCNC$H_2$), 3.75 (2H, s, ArC$H_2$), 7.15 - 7.25 (5H, m, Ar-*H*). $_c$ (75MHz, $CDCl_3$, $Me_4Si$): 26.9, 27.3, 30.2, 33.8, 42.2, 49.4, 54.1, 126.7, 127.9, 128.2, 140.4.

EXAMPLE II

Preparation of *N*-benzyl-*N'*-ferrocenylmethyl-hexamethylene-1, 6-diamine (**3**)

A solution of **2** (600mg, 2.91mmol) and ferrocenecarboxyaldehyde (747mg, 3.49mmol) in THF and methanol (15ml each) was stirred at room temperature for 7h under nitrogen atmosphere. Sodium borohydride (396mg, 10.5mmol) was added to this solution and then it was stirred at room temperature for 1h. The solvent was removed under reduced pressure, and the residue dissolved in chloroform. The chloroform phase was washed with water, and dried over magnesium sulphate and the solvent was removed under reduced pressure. The residue was purified by gel filtration {Sephadex LH-20, using methanol as eluent} to yield an orange oil (892mg, 76%).

$_H$ (300MHz, $CDCl_3$, $Me_4Si$) : 1.33 (4H, bs, NCC ($CH_2)_2$), 1.49 (4H, bs, NC($CH_2)_2$), 2.61 (2H, t, FcCNC$H_2$), 2.62 (2H, t, NC$H_2$), 3.50 (2H, s, NC$H_2$Fc), 3.78 (2H, s, PhC$H_2$N), 4. 09 - 4.12 and 4.18 (5H, 4H, m each, Fc-*H*), 7.34 (5H, m, Ar-*H*); $_c$ (75MHz, $CDCl_3$, $Me_4Si$) : 27.4, 30.2, 49.1, 49.5, 49.6, 54.1, 67.7, 68.3, 68.4, 126.7, 128.0, 128.2, 140.4.

EXAMPLE III

Preparation of N-benzyl-N, N'-bis(2-boronobenzyl)-N'-ferrocenylmethyl hexamethyl-1, 6-diamine (1).

A solution of 3 (800mg, 1.98mmol), potassium carbonate (1.10g, 7.92mmol), and 2-(2-bromobenzyl) 1,3,2-dioxaborinane (1.21g, 4.72mmol) in acetonitrile (35ml) was heated to reflux for 7h under a nitrogen atmosphere. The solvent was removed under reduced pressure, and the residue dissolved in chloroform. The chloroform phase was washed with water and dried over magnesium sulphate, and the solvent was removed under reduced pressure. The residue was reprecipitated from chloroform and n-hexane to yield a yellow powder (920mg, 69%).
mp 155-158°C (decomp); *m*/*z* (FAB) 1212 ({M+H+4(3-$HOCH_2C_6NO_2$)-4$H_2O$}$^+$, 95%); Found: C, 69.25; H, 7.07; N, 4.12%. $C_{38}H_{46}B_2FeN_2O_4$-$H_2O$+0.05 $CHCl_3$ requires C, 69.21; H, 6.74; N, 4.24%. $_H$ (300MHz, $CDCl_3$ + $CD_3OD$ (a few drops), $Me_4Si$) : 1.28 (4H, bs, NCC($CH_2)_2$), 1.42 (4H, bs, NC($CH_2)_2$), 2.28 (2H, t, FcCNC$H_2$), 2.36 (2H, t, NC$H_2$), 3.56 (4H, s, NC$H_2$Ph), 3.58 (2H, s, FcC$H_2$N), 3.78 (2H, s, NC$H_2$Ph), 4.12 and 4.18 (5H, 4H, s each, Fc-H), 7.15-7.38, 7.86 (11H, 2H, m each, Ar-*H*). $_c$ (75MHz, $CDCl_3$ + $CD_3OD$ (a few drops), $Me_4Si$): 22.7, 24.7, 25.6, 27.0, 31.6, 51.2, 52.0, 57.1, 60.4, 61.3, 68.4, 68.5, 68.6, 68.9, 70.6, 127.2, 127.3, 127.5, 128.2, 128.5, 129.6, 129.7, 129.9, 130.6, 130.9, 136.2, 136.5, 141.7, 141.8.

EXAMPLE IV

Preparation of N-methyl ferrocenylmethylamine (5).

A solution of ferrocenecarboxaldehyde (700mg, 3.27mmol) and methylamine (2.0M in THF) (5.0ml, 10.0mmol) in THF (25ml) and methanol (30ml) was stirred at room temperature for 15h under a nitrogen atmosphere. Sodium borohydride (371mg, 9.81mmol) was added to the solution and it was stirred at room temperature for 1h. The solvent was removed out under reduced pressure and the residue dissolved in chloroform. The chloroform was washed with water, dried over magnesium sulphate. The solvent was removed under reduced pressure to yield a brown oil (647mg, 86%).
$_H$ (300MHz, $CDCl_3$, $Me_4Si$) : 2.48 (3H, s, NC$H_3$), 3.47 (2H, s, NC$H_2$), 4 .12 - 4.18 (9H, m, Fc-*H*) $_{; c}$ (75MHz, $CDCl_3$, $Me_4Si$) : 36.6, 51.5, 68.1, 68.3, 68.7, 68.8, 69.2, 69.6, 69.8, 70.8, 87.1.

EXAMPLE V

Preparation of N- (2-borinobenzyl)-*N*-methyl ferrocenyl methylamine (**4**).

A solution of **5** (250mg, 1.09mol), potassium carbonate (301mg, 2.18mmol) and 2-(2-bromobenzyl)-1,3,2-dioxaborinane (333mg, 1.31mmol) in acetonitrile (10ml) was heated to reflux for 5h under a nitrogen atmosphere. The solvent was removed under reduced pressure, and the residue was dissolved in chloroform. The chloroform phase was washed with water, dried over magnesium sulphate. The solvent was removed under reduced pressure, the residue was reprecipitated from chloroform and n-hexane to yield a yellow powder (167mg, 42%)
mp 130-134°C (decomp) ; m/z (FAB) 633 ({M+H+2(3-$HOCH_2C_6H_4NO_2$)-2$_{H2}$O}$^+$, 58%) ; $_H$ (300MHz, $CDCl_3$ + $CD_3OD$ (a few drops), $Me_4Si$) : 2.23 (3H, s, NC$H_3$), 3.40 (2H, s, NC$H_2$Fc), 3.51(2H, s, NC$H_2$), 4.12 - 4.18 (9H, m, Fc-*H*), 6.72-7.28 (4H, m, Ar-*H*); $_c$ (75MHz, $CDCl_3$ + $CD_3OD$ (a few drops), $Me_4Si$) : 41.2, 55.4, 56.2, 68.7, 68.9, 69.1, 70.5, 114.7, 115.0, 117.0, 119.5, 127.4, 128.8.

EXAMPLE VI

Preparation of N-methyl benzylamine (7).

A solution of benzaldehyde (2.0ml, 20.0mmol) and methylamine (2.0M in THF) (20.0ml, 40.0mmol) and methanol (20ml) was stirred at room temperature for 20h under a nitrogen atmosphere. Sodium borohydride (2.27g, 60.0mmol) was added to the solution and it was stirred at room temperature for 2h. The solvent was removed out under reduced pressure and the residue dissolved in chloroform. The chloroform was washed with brine, and dried over magnesium sulphate. The solvent was removed under reduced pressure to yield a yellow oil (1.32g, 55%).
$_H$ (300MHz, $CDCl_3$, $Me_4Si$) : 2.42 (3H, s, NC$H_3$), 3.74 (2H, s, NC$H_2$Fc), 7.20-7.38 (5H, m, Ar-*H*) ; $_c$ (75MHz, $CDCl_3$,

$Me_4Si$) : 36.0, 56.1, 127.0, 128.4, 129.1, 140.1.

EXAMPLE VII

Preparation of N- (2-borinobenzyl)-N-methyl benzylamine (6).

A solution of 7 (500mg, 4.13mmol), potassium carbonate (1.14mg, 8.26mmol) and 2-(2-bromobenzyl)-1,3,2-dioxaborinane (1.26mg, 4.96mmol) in acetonitrile (20ml) was heated to reflux for 8h under a nitrogen atmosphere. The solvent was removed under reduced pressure, and the residue was dissolved in chloroform. The chloroform phase was washed with brine, and dried over magnesium sulphate. The solvent was removed under reduced pressure, the residue was reprecipitated from chloroform and n-hexane to yield a yellow powder (105mg, 10%)
mp 99-103°C; $_H$ (300MHz, $CD_3OD$, $Me_4Si$) : 2.71 (3H, s, $NCH_3$), 4.75 (2H, s, $NCH_2$), 4.80 (2H, s, $ArBNCH_2$), 7.10-7.68 (9H, m, Ar-*H*); $_c$ (75MHz, $CD_3OD$, $Me_4Si$): 38.8, 58.8, 61.9, 125.7, 126.9, 128.1, 128.4, 129.0, 129.1, 130.4, 133.1, 135.1, 135.8

EXAMPLE VIII

Differential pulse voltammogram (DPV).

The DPV shown in Figure 4 illustrates Differential Pulse Voltammogram (DPV) curves of compound 1 in the presence of concentrations of glucose ranging from 0-0.1 mol $dm^{-3}$. The D-glucose concentration for each of the curves from top to bottom are the following: 0 / mol $dm^{-3}$, $1.1x10^{-4}$ / mol $dm^{-3}$, $3.9x10^{-4}$ / mol $dm^{-3}$, $6.7x10^{-4}$ / mol $dm^{-3}$, $1.0x10^{-3}$ / mol $dm^{-3}$, $3.8x10^{-3}$ / mol $dm^{-3}$, $6.0x10^{-3}$ / mol $dm^{-3}$, and $1.0x10^{-1}$ / mol $dm^{-3}$.

The measurement solution for the differential pulse voltammograms (DPV) was a aqueous methanolic buffer solution comprising 52.1wt% methanol at pH 8.21, KCl, 0.01000 mol $dm^{-3}$; $KH_2PO_4$, 0.002752 mol $dm^{-3}$; and $Na_2HPO_4$ 0.002757 mol $dm^{-3}$. The measurements were preformed with 30ml of the measurement solution in the measurement cell under nitrogen. The measurement temperature was room temperature (25°C). The scanning conditions were the following: modulation time, 50ms; interval time, 500ms; step potential, 5.1mV; modulation amplitude, 25.05mV; initial potential, 0.1V; end potential, 0.6V.

The DPV was recorded with AUTOLAB Type II (AUTOLAB Co.) using single-compartment cell fitted with a glassy carbon electrode for working electrode (0.28 $cm^{-2}$), a platinum plate for counter electrode, and a Ag / AgCl reference electrode. The scanning conditions were modulation time, 50ms; interval time, 500ms; step potential, 5.1mV; modulation amplitude 25.05mV. The voltammogram, current intensity at 0.372 volt decreases with increasing saccharide concentration and a 0.450 volt species due to the bound species appears. The stability constant K with each saccharide were calculated by curve fitting the current intensity at 0.372 volt versus concentration of saccharide.

Although the present invention has been described in considerable detail with reference to certain preferred versions thereof, other versions are possible. Therefore, the spirit and scope of the appended claims should not be limited to the description of the preferred versions described herein.

## Claims

1. A compound comprising:

    a first boronic acid group attached by a first linker group to a first amine group, wherein the first amine group is attached to a reporter group comprising an organometallic reporter moiety; and
    a second boronic acid group attached by a second linker group to a second amine group, wherein the second amine group is attached to an R group, wherein the R group is selected from the group consisting of hydrogen, alkyl groups, aryl groups, and a reporter group comprising an organometalic reporter moiety; and
    a spacer group attaching the first amine group to the second amine group.

2. A compound according to Claim 1, wherein the first linker group is selected from the group consisting of aromatic rings, heteroaromatic rings, alkyl groups, alkene groups, and alkyne groups.

3. A compound according to Claim 1 or 2 wherein the spacer group is an alkyl group.

4. A compound according to any one of the preceding Claims wherein the spacer group is a hexamethylene group.

5. A compound according to any one of the preceding Claims, wherein the second linker group is selected from the group consisting of aromatic rings, heteroaromatic rings, alkyl groups, alkene groups, and alkyne groups.

6. A compound according to any one of the preceding Claims, wherein the organometallic reporter moiety is ferrocene or a ferrocene derivative and the compound binds and detects saccharides ectrochemically.

7. A compound according to Claim 1 of the formula:

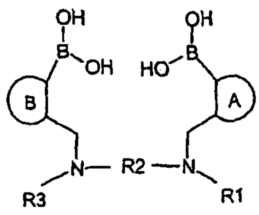

wherein
A is the first linker group, which is an aromatic ring or a heteroaromatic ring;
R1 is the reporter group comprising an organometallic reporter group;
R2 is the linker group, which is an alkyl group or an aryl group;
R3 is the R group, which is hydrogen, an alkyl group, an aryl group, or an organometallic reporter group; and
B is the second linker group, which is an aromatic ring or heteroaromatic ring.

8. A compound according to Claim 7 for detecting glucose of the formula:

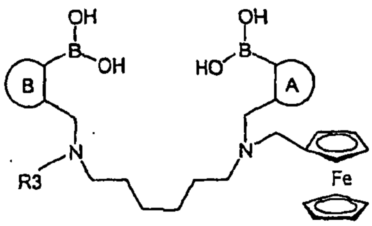

wherein A, B, and R3 are defined as in Claim 7.

9. A compound according to Claim 1 of the formula:

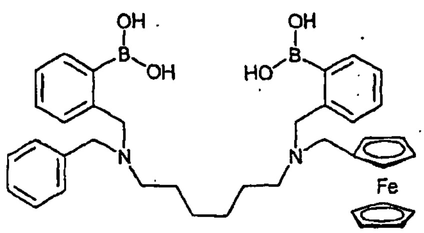

10. A compound according to any of the preceding Claims, wherein the compound has a higher relative stability constant for glucose than for other saccharides.

11. A compound according to any of the preceding Claims, wherein the compound has a relative stability constant for glucose that is at least two times higher than the relative stability constant for the compound and other saccharides.

12. A compound according to any one of the preceding Claims, wherein the compound has a relative stability constant for glucose that is between about two and about fifteen times higher than the relative stability constant for the

compound and other saccharides.

**13.** A method for detecting saccharides in a sample comprising:

providing a predetermined amount of a compound according to any one of the preceding Claims;
treating the sample with the compound; and
detecting saccharides bound to the compound.

**14.** A method according to Claim 13 wherein the method detects glucose relative to other saccharides.

**15.** A method according to Claim 13, wherein the compound is

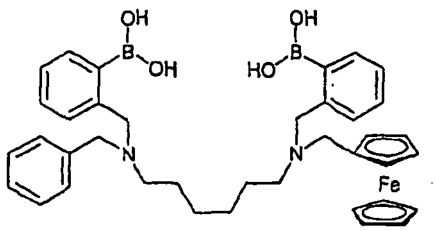

and the method detects glucose relative to other saccharides.

**Patentansprüche**

**1.** Verbindung, umfassend:

eine erste Boronsäuregruppe, gebunden über eine erste Verknüpfungsgruppe zu einer ersten Amingruppe, wobei die erste Amingruppe an eine Reportergruppe, umfassend eine organometallische Reportereinheit, gebunden ist, und
eine zweite Boronsäuregruppe, gebunden über eine zweite Verbindungsgruppe zu einer zweiten Amingruppe, wobei die zweite Amingruppe an eine Gruppe R gebunden ist, wobei die Gruppe R aus der Gruppe, bestehend aus Wasserstoff, Alkylgruppen, Arylgruppen und einer Reportergruppe, umfassend eine organometallische Reportereinheit, ausgewählt ist, und
eine Spacergruppe, welche die erste Amingruppe an die zweite Amingruppe bindet.

**2.** Verbindung gemäß Anspruch 1, wobei die erste Verknüpfungsgruppe aus der Gruppe, bestehend aus aromatischen Ringen, heteroaromatischen Ringen, Alkylgruppen, Alkengruppen und Alkingruppen, ausgewählt ist.

**3.** Verbindung gemäß Anspruch 1 oder 2, wobei die Spacergruppe eine Alkylgruppe ist.

**4.** Verbindung gemäß einem der vorhergehenden Ansprüche, wobei die Spacergruppe eine Hexamethylengruppe ist.

**5.** Verbindung gemäß einem der vorhergehenden Ansprüche, wobei die zweite Verknüpfungsgruppe aus der Gruppe, bestehend aus aromatischen Ringen, heteroaromatischen Ringen, Alkylgruppen, Alkengruppen und Alkingruppen, ausgewählt ist.

**6.** Verbindung gemäß einem der folgenden Ansprüche, wobei die organometallische Reportereinheit Ferrocen oder ein Ferrocenderivat ist, und die Verbindung Saccharide bindet und electrochemisch detektiert.

**7.** Verbindung gemäß Anspruch 1 gemäß der Formel:

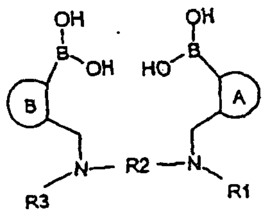

worin

A die erste Verknüpfungsgruppe ist, welche ein aromatischer oder heteroaromatischer Ring ist,
R1 die Reportergruppe, umfassend eine organometallische Reportergruppe, ist,
R2 die Verknüpfungsgruppe ist, welche eine Alkylgruppe oder eine Arylgruppe ist,
R3 die Gruppe R ist, welche Wasserstoff, eine Alkylgruppe, eine Arylgruppe oder eine organometallische Reportergruppe ist, und
B die zweite Verknüpfungsgruppe ist, welche ein aromatischer Ring oder ein heteroaromatischer Ring ist.

**8.** Verbindung gemäß Anspruch 7 zum Detektieren von Glucose gemäß der Formel

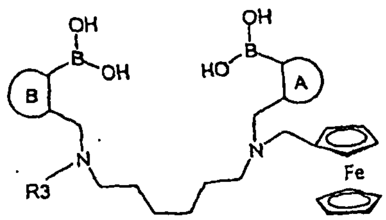

worin A, B und R3 wie in Anspruch 7 definiert sind.

**9.** Verbindung gemäß Anspruch 1 gemäß der Formel:

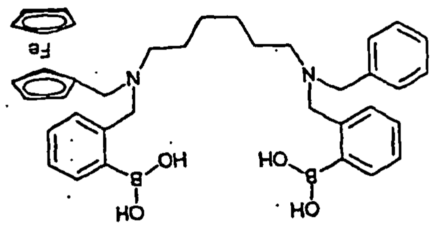

**10.** Verbindung gemäß einem der vorhergehenden Ansprüche, wobei die Verbindung eine höhere relative Stabilitätskonstante für Glucose als für andere Saccharide aufweist.

**11.** Verbindung gemäß einem der vorhergehenden Ansprüche, wobei die Verbindung eine relative Stabilitätskonstante für Glucose aufweist, die mindestens zweimal größer als die relative Stabilitätskonstante für die Verbindung und andere Saccharide ist.

**12.** Verbindung gemäß einem der vorhergehenden Ansprüche, wobei die Verbindung eine relative Stabilitätskonstante für Glucose aufweist, die zwischen etwa dem zwei- und etwa dem 15-fachen größer als die relative Stabilitätskonstante für die Verbindung und andere Saccharide ist.

**13.** Verfahren zum Detektieren von Sacchariden in einer Probe, umfassend:

das Bereitstellen einer vorbestimmten Menge einer Verbindung gemäß einem der vorhergehenden Ansprüche,
das Behandeln der Probe mit der Verbindung, und
das Detektieren von Sacchariden, gebunden an die Verbindung.

**14.** Verfahren gemäß Anspruch 13, wobei das Verfahren Glucose, relativ zu anderen Sacchariden, detektiert.

**15.** Verfahren gemäß Anspruch 13, wobei die Verbindung

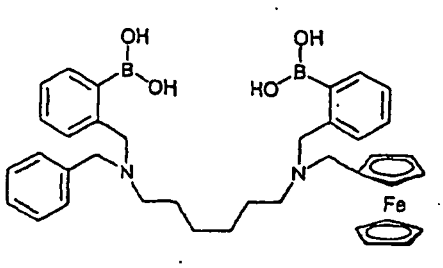

ist und das Verfahren Glucose, relativ zu anderen Sacchariden, detektiert.

## Revendications

**1.** Composé comprenant :

un premier groupe d'acide boronique lié par un premier groupe de liaison à un premier groupe amine, le premier groupe amine étant lié à un groupe reporter comprenant un fragment reporter organométallique ; et
un deuxième groupe d'acide boronique lié par un deuxième groupe de liaison à un deuxième groupe amine, le deuxième groupe amine étant lié à un groupe R, le groupe R étant choisi dans le groupe comprenant l'hydrogène, des groupes alkyle, des groupes aryle et un groupe reporter comprenant un fragment reporter organométallique ; et
un groupe d'espacement liant le premier groupe amine au deuxième groupe amine.

**2.** Composé selon la revendication 1, dans lequel le premier groupe de liaison est choisi dans le groupe comprenant des cycles aromatiques, des cycles hétéroaromatiques, des groupes alkyle, des groupes alcène et des groupes alcyne.

**3.** Composé selon la revendication 1 ou 2, dans lequel le groupe d'espacement est un groupe alkyle.

**4.** Composé selon l'une quelconque des revendications précédentes, dans lequel le groupe d'espacement est un groupe hexaméthylène.

**5.** Composé selon l'une quelconque des revendications précédentes, dans lequel le deuxième groupe de liaison est choisi dans le groupe comprenant des cycles aromatiques, des cycles hétéroaromatiques, des groupes alkyle, des groupes alcène et des groupes alcyne.

**6.** Composé selon l'une quelconque des revendications précédentes, dans lequel le fragment reporter organométallique est un ferrocène ou un dérivé de ferrocène et le composé se lie et détecte électrochimiquement des saccharides.

**7.** Composé, selon la revendication 1, de formule :

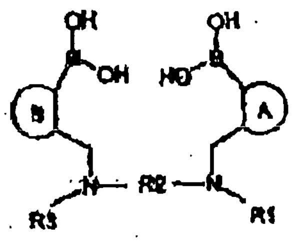

dans laquelle
A est le premier groupe de liaison, qui est un cycles aromatique ou un cycle hétéroaromatique ;
R1 est un groupe reporter comprenant un groupe reporter organométallique ;
R2 est le groupe de liaison qui est un groupe alkyle ou un groupe aryle ;
R3 est le groupe R qui est l'hydrogène, un groupe alkyle, un groupe aryle ou un groupe reporter organométallique ; et
B est le deuxième groupe de liaison qui est un cycle aromatique ou un cycle hétéroaromatique.

**8.** Composé selon la revendication 7, pour détecter le glucose, de formule :

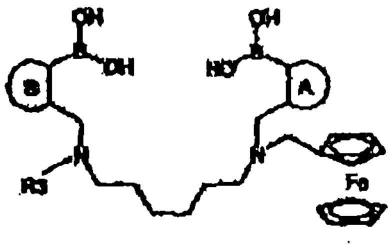

dans laquelle A, B et R3 sont tels que définis à la revendication 7.

**9.** Composé selon la revendication 1, de formule :

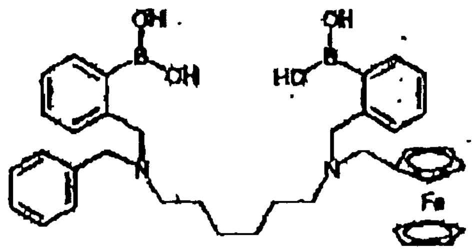

**10.** Composé selon l'une quelconque des revendication précédentes, le composé ayant une constante de stabilité relativement supérieure du glucose, par rapport à celle d'autres saccharides.

**11.** Composé selon l'une quelconque des revendications précédentes, le composé ayant une constant de stabilité relative pour le glucose qui est au moins deux fois supérieure à la constante de stabilité relative du composé et autres saccharides.

**12.** Composé selon l'une quelconque des revendications précédentes, le composé ayant une constante de stabilité relative du glucose qui est entre environ deux et environ quinze fois supérieure à la constante de stabilité relative du composé et autres saccharides.

**13.** Procédé de détection de saccharides dans un échantillon comprenant :

la fourniture d'une quantité prédéterminée d'un composé selon l'une quelconque des revendications précédentes ;
le traitement de l'échantillon avec le composé; et
la détection de saccharides liés au composé.

**14.** Procédé selon la revendication 13, le procédé détectant le glucose par rapport aux autres saccharides.

**15.** Procédé selon la revendication 13, le composé étant

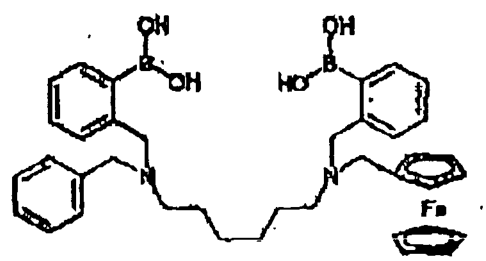

et le procédé détectant le glucose par rapport à d'autres saccharides.

**Figure 1**

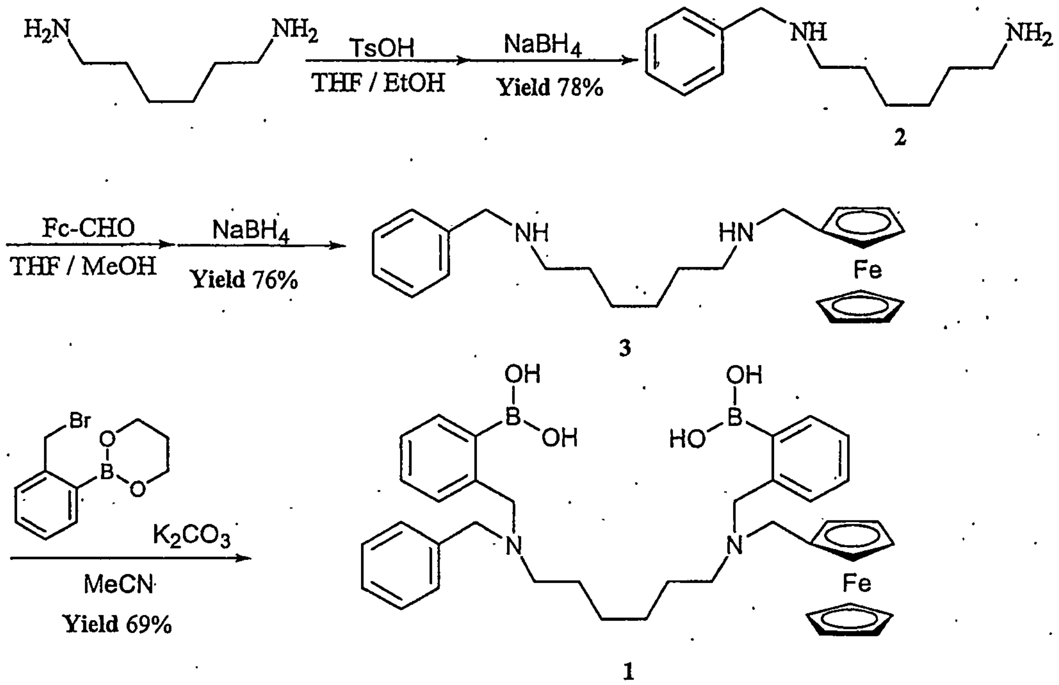
TsOH
THF / EtOH
NaBH4
Yield 78%
2
Fc-CHO
THF / MeOH
NaBH4
Yield 76%
3
K2CO3
MeCN
Yield 69%
1

· Figure 2

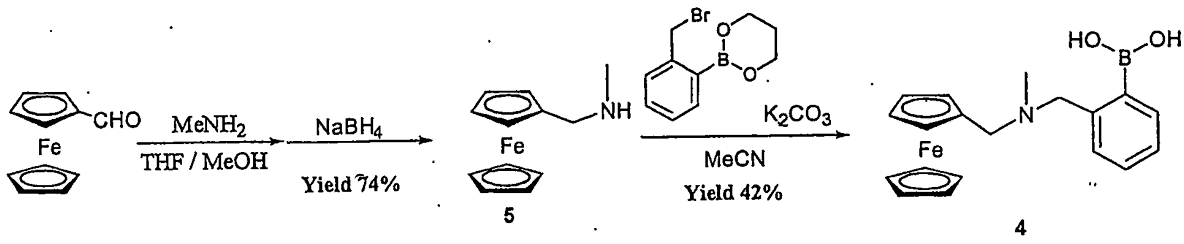

Figure 3

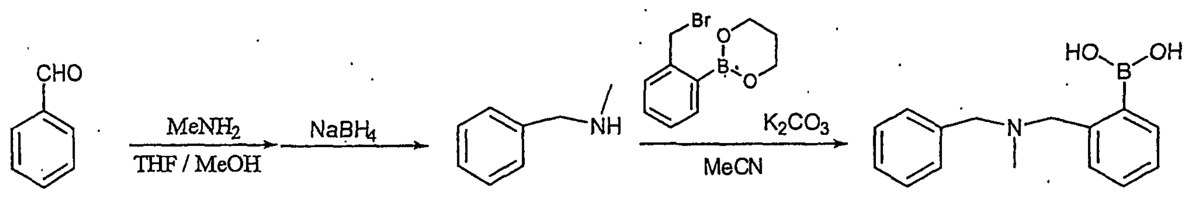


7

6

Figure 4

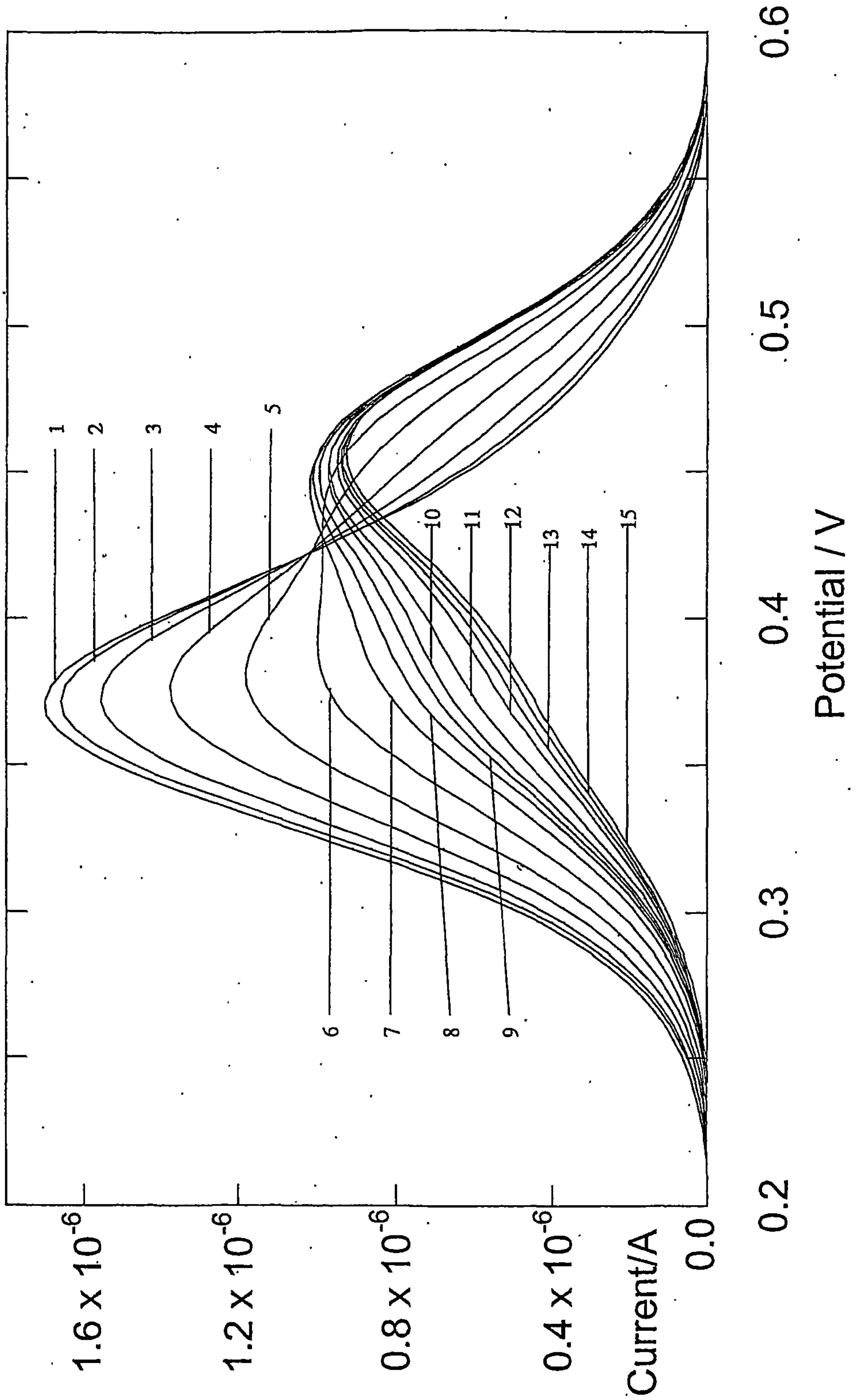

Current/A
0.0
0.4 x 10-6
0.8 x 10-6
1.2 x 10-6
1.6 x 10-6
Potential / V
0.2
0.3
0.4
0.5
0.6
1
2
3
4
5
6
7
8
9
10
11
12
13
14
15

Figure 5

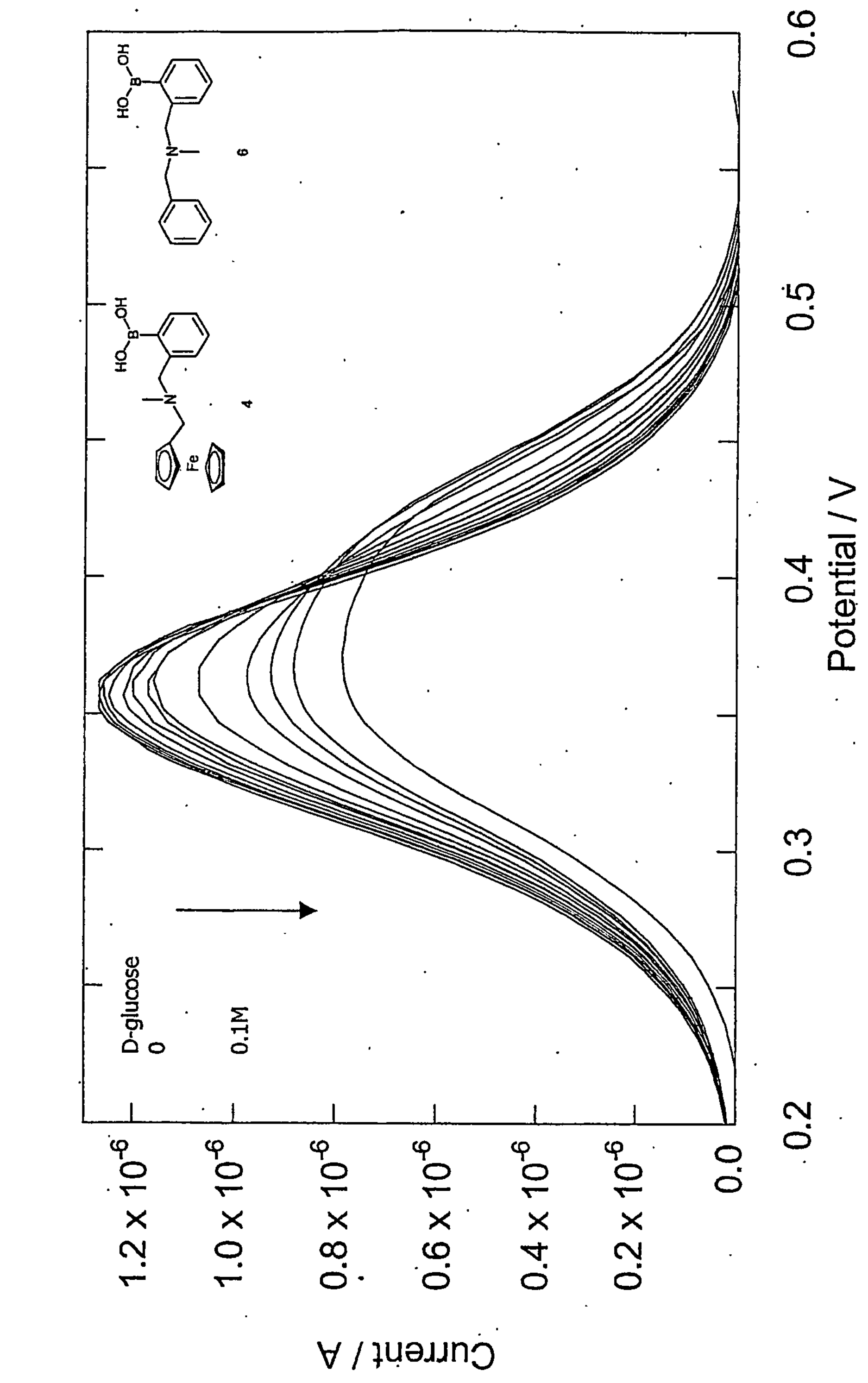
D-glucose
0
0.1M
4
6
Current / A
1.2 x 10^-6
1.0 x 10^-6
0.8 x 10^-6
0.6 x 10^-6
0.4 x 10^-6
0.2 x 10^-6
0.0
0.2
0.3
0.4
0.5
0.6
Potential / V

Figure 6

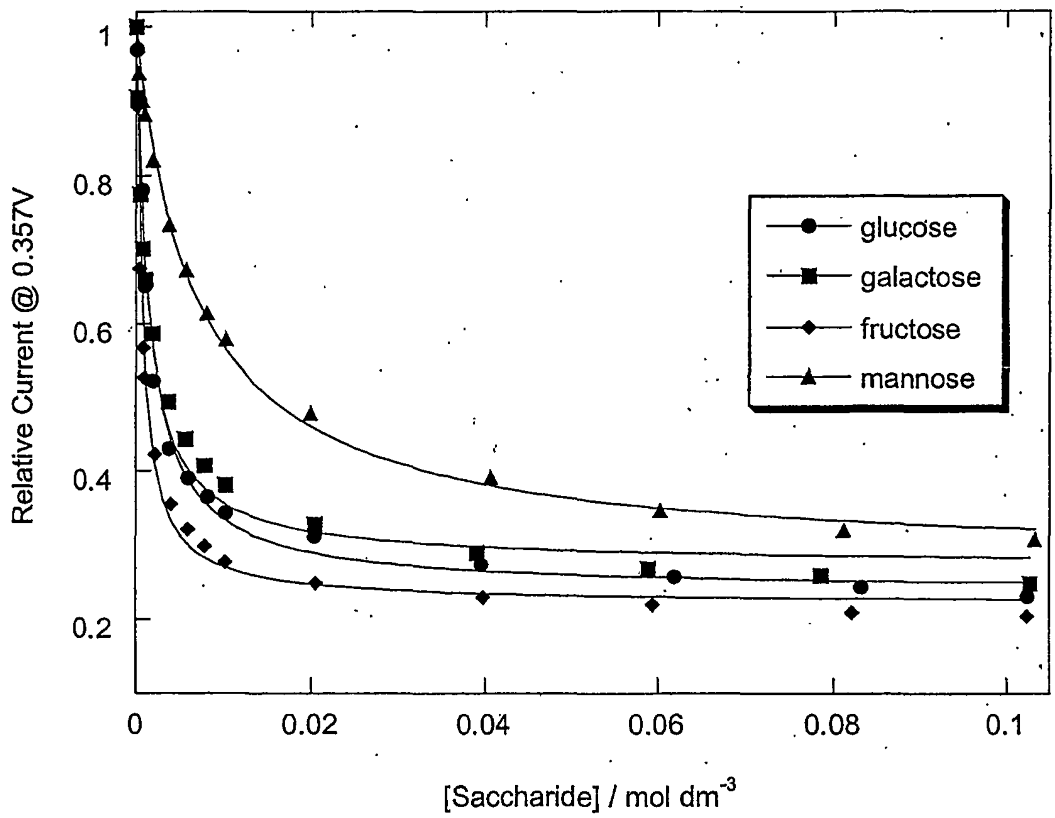
1
0.8
0.6
0.4
0.2
Relative Current @ 0.357V
0
0.02
0.04
0.06
0.08
0.1
[Saccharide] / mol dm-3
glucose
galactose
fructose
mannose

Figure 7

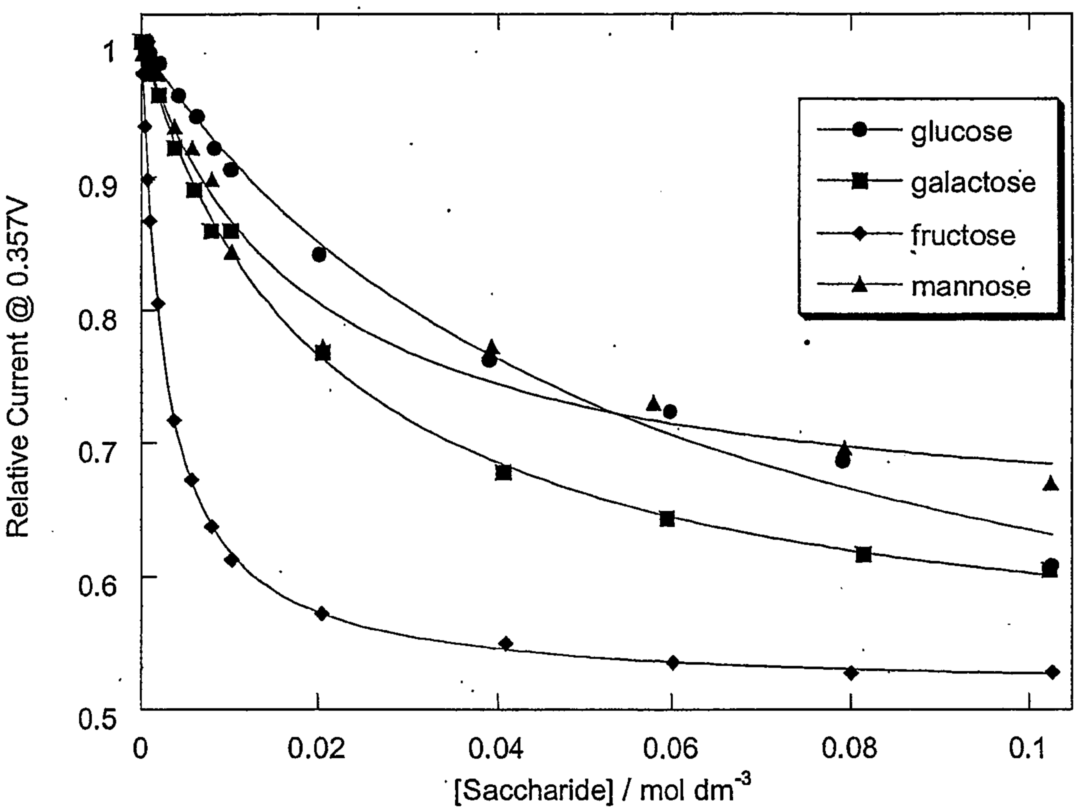
glucose
galactose
fructose
mannose
Relative Current @ 0.357V
[Saccharide] / mol dm-3
1
0.9
0.8
0.7
0.6
0.5
0
0.02
0.04
0.06
0.08
0.1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6387672 A

### Non-patent literature cited in the description

- **A.N.J. MOORE ; D. D. M. WAYNER.** Redox switching of carbohydrate binding to ferrocene boronic acid. *Canadian Journal of Chemistry-Revue Canadienne De Chimie,* 1999, vol. 77, 681
- **J. P. LORAND ; J. D. EDWARD.** Polyol complexes and structure of the benzeneboronate ion. *Journal of Organic Chemistry,* 1959, vol. 24, 769
- **C. M. A. BRETT ; A. M. OLIVIERA BRETT.** Electrochemistry. Oxford university press, 1993
- **FERY-FORGUES, S. et al.** Ion-responsive fluorescent compounds. 1. Effect of cation binding on photophysical properties of a benzoxazione derivative linked to monoaza-15-crown-5. *J. Phys. Chem.,* 1988, vol. 92, 6233-6237